# EUROPEAN PATENT APPLICATION

(11) **EP 1 726 657 A1**
(43) Date of publication of application: **29.11.2006**
(21) Application number: 05710043.0
(22) Date of filing: 03.02.2005
(51) Int. Cl.: C12N 15/87

(54) **METHOD OF CONVERTING BASE IN DNA SEQUENCE**

(30) Priority: 10.02.2004 JP 2004034019
(71) Applicant: Japan Science and Technology Agency, Kawaguchi-shi, Saitama 332-0012 (JP)
(72) Inventor: KAMIYA, Hiroyuki, Kita-10-Jo-Gurandohaitsu 402, Sapporo-shi, Hokkaido 001-0010 (JP); HARASHIMA, Hideyoshi, Sapporo-shi, Hokkaido 001-0907 (JP); TSUCHIYA, Hiroyuki, Sapporo-shi, Hokkaido 001-0013 (JP)
(74) Representative: Owen, Deborah Jane
(86) International application number: PCT/JP2005/001991
(87) International publication number: WO 2005/075657

(57) **Abstract**

A method of converting one or more bases in a target DNA sequence in a cell comprising transferring a single-stranded DNA fragment having 300 to 3,000 bases, which is prepared from a single-stranded circular DNA, is homologous with the target DNA sequence and contains the base(s) to be converted, into a cell.

## Description

### Technical Field

The invention of this application relates to a method of converting a base in a DNA sequence. More particularly, the invention of this application relates to a method of converting one or more bases or a base sequence in a target DNA sequence in a cell to another base or a base sequence.

### Background Art

As a result of recent progress of the human genome project or the development of screening methods for a large amount of gene samples, it became possible to investigate individual gene information in more detail and simply (documents 1 and 2). Along with the progress of such techniques, a personalized gene correction method in which a sequence with a mutation is directly returned to a normal sequence in accordance with the gene information of an individual patient is a very promising technique in gene therapy. The small fragment homologous replacement (SFHR) method, which is one of the gene correction methods, intends to convert a mutant gene to a normal form by introducing a thermally denatured PCR product (a double-stranded DNA fragment) containing a normal gene sequence into a cell (documents 3 to 5). It has been reported that a causative gene for such as cystic fibrosis or muscular dystrophy can be corrected by this method (documents 6 and 7). It can be expected that a gene corrected in this way is appropriately expressed in a cell under the regulation of the original promoter in the same manner as a normal gene. Further, it is also possible to treat or correct a gain-of-function mutant typified by an oncogene, which has been considered to be difficult to treat or repair by the current gene therapy in principle.

Further, as a gene correction method using a single-stranded DNA fragment, a method using an end-modified oligonucleotide obtained by chemical synthesis is known. Regarding this method, it has been reported that a correction efficiency of 10⁻² and 10⁻⁵ is exhibited in a mammalian cell and a yeast cell, respectively, using a single-stranded DNA having 25 to 100 bases modified at both ends with phosphorothioate (documents 8 to 10), 2'-O-Me-RNA (documents 11 to 13) or LNA (locked nucleic acid) (document 14). Further, in the method using this end-modified oligonucleotide, it is known that a higher gene correction efficiency is exhibited when a single-stranded DNA fragment homologous with an antisense strand of a target DNA sequence is used (documents 8 and 11).

As described above, the SFHR method is extremely promising as a new form of gene therapy. However, the current correction efficiency of mutant gene in the SFHR method stays less than 1%, although it greatly varies depending on the assay system to be used or the target gene, and it is essential to dramatically improve this value in order to make the SFHR method a clinically applicable technique.

Accordingly, the invention of this application has been made in view of the above-mentioned circumstances, and an object of the invention is to provide a method capable of preparing a single-stranded DNA fragment for gene correction without separating it from a complementary strand and also capable of more efficiently converting a base in a DNA sequence in a cell utilizing the resulting single-stranded DNA fragment.

### Documents

Document 1: Joos L, Eryusel E and Brutsche MH. Functional genomics and gene microarrays - the use in research and clinical medicine. Swiss Med Wkly. 2003; 133:31-38
Document 2: Ferrari M, Stenirri S, Bonini P, Cremonesi L. Molecular diagnostics microelectronic microchips. Clin Chem Lab Med. 2003; 41:462-467
Document 3: Kunzelmann K. Legendre J-Y, Knoell DL, Escobar LC, Xu Z and Gruenert DC. Gene targeting of CFTR DNA in CF epitherial cells. Gene Ther. 1996; 3:859-867
Document 4: Goncz KK, Kunzelmann K, Xu Z and Gruenert DC. Targeted replacement of normal and mutant CFTR sequences in human airway epithelial cells using DNA fragments Hum Mol Genet. 1998; 7:1913-1919
Document 5: Colosimo A, Goncz KK, Novelli G, Dallapiccola B and Gruenert DC. Targeted correction of a defective selectable marker gene in human epithelial cells by small DNA fragments. Mol Ther. 2001; 3:178-185
Document 6: Goncz KK, Colosimo A, Dallapiccola B, Gagne L, Hong K, Novelli G, Papahadjopoulos D, Sawa T, Schreier H, Weiner-Kronish J, Xu Z and Gruenert DC. Expresion of delta-F508 CFTR in normal mouse lung after site-specific modification of CFTR sequences by SFHR. Gene Ther. 2001; 8:961-965
Document 7: Kapsa R, Quigley A, Lynch GS, Steeper K, Kornberg AJ, Gregorevic P, Austin L and Byrne E, In vivo and in vitro correction of the mdx dystrophin gene nonsense mutation by short-fragment homologous replacement. Hum Gene Ther. 2001; 12:629-642
Document 8: Liu L, Rice MC, Drury M, Cheng S, Gamper H and Kmiec EB. Strand bias in targeted gene repair is influenced by transcriptional activity. Mol Cell Biol. 2002; 22:3852-3863
Document 9: Lin L, Cheng S. van Brabant AJ and Kmiec EB. Rad51p and Rad54p, but not Rad52p, elevate gene repair in Saccharomyces cerevisiae directed by modified single-stranded oligonucleotide vectors. Nuclic Acids Res. 2002; 30:2742-2750
Document 10: Brachman EE and Kmiec EB. targeted nucleotide repair of cycl mutations in Saccharomyces cerevisiae directed by modified single-stranded nucleotides. Genetics. 2003; 163:527-538
Document 11: Igoucheva O, Alexeev V and Yoon K. Targeted gene correction by small single-stranded oligonucleotides in mammalian cells. Gene Ther. 2001; 8:391-399
Document 12: Alexeev V, Igoucheva O and Yoon K. Simultaneous targeted alteration of the tyrosinase and c-kit genes by single-stranded oligonucleotides. Gene Ther. 2002; 9:1667-1675
Document 13: Pierce EA, Liu Q, Igoucheva O, Omarrudin R, Ma H, Diamond SL and Yoon K. Oligonucleotide-directed single base DNA alterations in mouse embryonic stem cells. Gene Ther. 2003; 10:24-33
Document 14: Parekh-Olmedo H, Drury M and Kmiec EB. Targeted nucleotide exchange in Saccharomyces cerevisiae directed by short oligonucleotides containing locked nucleic acids. Chem Biol. 2002; 9:1073-1084

### Disclosure of the invention

In this application, a first invention for achieving the above objects is a base conversion method of a DNA sequence, which is a method of converting one or more bases in a target DNA sequence in a cell, characterized by introducing a single-stranded DNA fragment having 300 to 3,000 bases which is prepared from a single-stranded circular DNA, is homologous with the target DNA sequence, and contains the base(s) to be converted, into a cell.

In this first invention, a preferred embodiment is that the single-stranded DNA fragment is homologous with a sense strand of the target DNA sequence.

Further, one embodiment in this first invention is that the single-stranded circular DNA is a phagemid DNA, and base conversion is carried out for the target DNA sequence which is a cause of a disease due to a mutation of one or more bases.

Further, another embodiment in this first invention is that one or more bases in a target DNA sequence in a cell of an organism are converted.

A second invention of this application is a cell in which one or more bases in a target DNA sequence have been converted by the method of the above-mentioned first invention.

A third invention of this application is an individual organism which retains the cell of the above-mentioned second invention in the body.

A fourth invention of this application is a therapeutic agent, which is an agent for treating a disease caused by a mutation of one or more bases in a target DNA sequence, having a form such that a single-stranded DNA fragment having 300 to 3,000 bases which is prepared from a single-stranded circular DNA, is homologous with the target DNA sequence, and contains the base(s) to be converted, can be introduced into a cell.

One embodiment in this fourth invention is that the single-stranded circular DNA is a phagemid DNA.

A fifth invention of this application is a therapeutic method, which is a method of treating a disease caused by a mutation of one or more bases in a target DNA sequence, characterized by introducing a single-stranded DNA fragment having 300 to 3,000 bases which is prepared from a single-stranded circular DNA, is homologous with the target DNA sequence, and contains the base(s) to be converted, into a cell. Further, one embodiment in the fifth invention is that the single-stranded circular DNA is a phagemid DNA.

Incidentally, in this invention, a "DNA sequence" means a molecule obtained by binding phosphate esters of a nucleoside (dATP, dGTP, dCTP and dTTP) in which a purine or a pyrimidine has been bound to a sugar through a β-N-glycoside bond.

Further, "conversion" means replacement of one or more bases (A, T, C and G) in a target DNA sequence with another base, respectively (base substitution), deletion of one or more bases in a target DNA sequence (base deletion), and addition of one or more bases to a target DNA sequence (base addition). Further, such base conversion may target one or more bases which are individually independent in a target DNA sequence, or substitution, deletion or addition of a sequence composed of a plurality of bases (sequence substitution, sequence deletion, sequence addition, respectively: hereinafter these are described as "sequence conversion" in some cases) in a target sequence.

The other terms and concepts in this invention will be defined in detail in the description of the embodiments or Example of the invention. In addition, various techniques to be used for implementing this invention can be easily and surely carried out by a person skilled in the art based on known literatures and the like except for the techniques whose sources are particularly specified. For example, preparation of a therapeutic agent is described in Remington's Pharmaceutical Sciences, 18th Edition, ed. A. Gennaro, Mack Publishing Co., Easton, PA, 1990, Sambrook and Maniatis, Molecular Cloning-a Laboratory Manual, Cold Spring Harbor Laboratory Press, New York, 1989; and Ausubel, F. M. et al., Current Protocols in Molecular Biology, John Wiley & Sons, New York, N.Y, 1995. The terms in this invention are basically based on IUPAC-IUB Commission on Biochemical Nomenclature, or the meanings of words conventionally used in this field.

According to the above-mentioned invention of this application as described above, a desired single-stranded DNA fragment for gene correction is prepared from a single-stranded circular DNA which does not have a complementary strand, therefore, it is not necessary to separate the desired single-stranded DNA fragment from the complementary strand, and moreover, compared with the conventional SFHR method, it becomes possible to accurately convert a base or a base sequence in a target DNA sequence with high efficiency. In particular, by using a single-stranded DNA fragment homologous with a sense strand of a target DNA sequence, it becomes possible to accurately convert a desired base in the target gene efficiently.

### Brief Description of the Drawings

Fig. 1A shows the constructs of target plasmids pTENHES and pTENHEX into which a normal form and a mutated form of HygEGFP genes have been integrated, respectively. Fig. 1B shows the constructs of phagemid vectors pBSHES/AntSense and pBSHES/Sense for preparing a single-stranded antisense DNA fragment and sense DNA fragment.
Fig. 2 shows photomicrographs for observing fluorescent signals in the case where a target plasmid pTENHEX and a single-stranded sense DNA fragment have been introduced into a mammalian cell (CHO-K1 cell). The mutated form of HygEGFP gene is corrected by the single-stranded DNA fragment and the EFGP gene is expressed, whereby a fluorescent signal is generated.

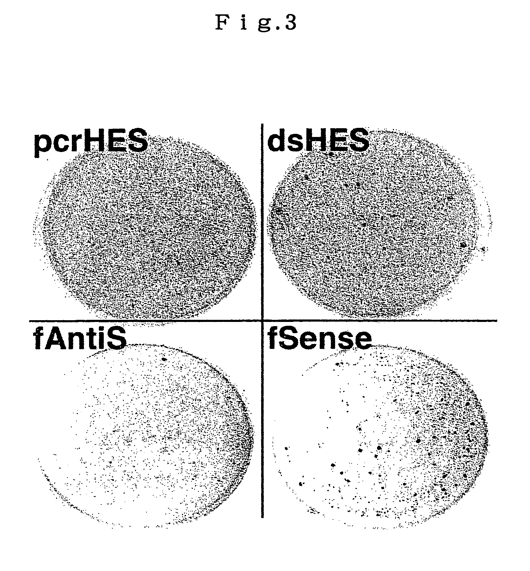

Fig. 3 shows images, analyzed with an image analyzer, of the state where a plasmid DNA recovered from a mammalian cell (CHO-K1 cell) into which the target plasmid pTENHEX and a single-stranded DNA fragment (fAntiS or fSense), a double-stranded DNA fragment (dsHES), and a PCR product (pcrHES) had been introduced was introduced into E. coli, and the E. coli was cultured on an agar medium supplemented with hygromycin B. In particular, when the single-stranded sense DNA fragment (fSense) is introduced, the mutated form of HygEGFP gene is corrected, and the cell acquires hygromycin B resistance and a fluorescent signal- positive trait.
Fig. 4 is a graph showing the gene correction efficiency of respective amounts of dsHES, fAntiS, fSense and pcrHES introduced into cells.
Fig. 5 shows the results of electrophoresis of the restricted enzyme PmaCI fragments of PCR products amplified using the target plasmid extracted from an EGFP-positive cell.
Fig. 6 is a chart showing the results of sequence analysis of the target plasmid extracted from an EGFP-positive cell.

### Best Mode for Carrying Out the Invention

The invention of this application has characteristic features as described above, however, hereinafter an embodiment thereof will be described in detail.

The method of the first invention is characterized in that one or more bases in a target DNA sequence are converted by introducing a single-stranded DNA fragment having 300 to 3,000 bases which is prepared from a single-stranded circular DNA, is homologous with the target DNA sequence, and contains the base(s) to be converted, into a cell. That is, the principle of this method is to convert a base or a base sequence to be converted in a target DNA sequence (hereinafter referred to as a "target base") to another base or base sequence (hereinafter referred to as a "conversion base") by "homologous replacement" of the target DNA sequence or a partial region thereof with a single-stranded DNA fragment. Further, while in the conventional SFHR method, a double-stranded DNA fragment (for example, a PCR product) is thermally denatured to form the respective single-stranded DNA fragments and both sense strand and antisense strand are allowed to act on a target DNA sequence, the method of this invention is characterized by allowing only a single-stranded DNA fragment (either a sense strand or an antisense strand, preferably a sense strand) to act on a target DNA sequence.

The "single-stranded DNA fragment" is homologous with a target DNA sequence except that it contains a conversion base. The "homologous" in this case means that a sequence is at least 95%, preferably at least 98%, more preferably at least 99%, and ideally 100% identical to either of the sense strand or the antisense strand of the double-stranded target DNA sequence, however, it is preferred that a sequence is homologous with the sense strand of a target DNA sequence in terms of the conversion efficiency.

Further, this single-stranded DNA fragment is not modified at its ends. That is, it is a DNA fragment in which both ends are not modified with phosphorothioate, 2'-O-Me-RNA or LNA (locked nucleic acid) like the above-mentioned modified oligonucleotide.

The "size of the single-stranded DNA fragment" is selected from the range of 300 to 3,000 bases according to the length of a target DNA sequence, the number of target bases or the like. For example, it is a DNA fragment having a length of 300 to 500 bases, 500 to 800 bases, 800 to 1200 bases, 1200 to 1700 bases, 1700 to 2300 bases, 2300 to 3000 bases or the like.

The "target DNA sequence" is not particularly limited as long as it is a DNA sequence present in a cell, and a DNA sequence that plays a role in some function in a cell such as a DNA sequence of a genomic gene, a DNA sequence of mitochondria or a DNA sequence of a plasmid can be used as a target.

The number of "conversion bases" in the single-stranded DNA fragment depends on the number of target bases in the target DNA sequence. For example, in the case of individual base conversion (base substitution, base deletion or base addition), depending on the size of the target DNA sequence or the single-stranded DNA fragment, the number can be determined to be one or more (2 to about 30 bases), however, in order to achieve efficient replacement, 1 to about 10 bases are preferred. Further, in the case of sequence conversion, a sequence composed of 2 to about 30 bases, preferably 2 to about 10 bases is determined to be the conversion base. Further, the position of the conversion base in the single-stranded DNA fragment is not particularly limited, however, it is preferred that the position is not an end portion of the DNA fragment.

The single-stranded DNA fragment can be prepared, for example, as follows. For example, in the case where the target DNA sequence is a sequence with a base mutation (for example, a genomic gene DNA sequence which is a cause of a disease), a single-stranded DNA fragment is preferably prepared by amplifying, for example, a normal genomic gene DNA sequence or cDNA thereof using an appropriate single-stranded DNA vector (for example, a phagemid DNA or the like), fragmenting the amplified product with an enzyme or the like and separating or purifying the target fragment from unnecessary fragments. According to such a preparation process, a single-stranded DNA fragment can be obtained without separating it from a complementary strand and whether or not it is a short strand or a long strand.

Incidentally, as the method of preparing a DNA fragment, a method of amplifying a template DNA in a test tube [for example, the PCR (polymerase chain reaction) method, the NASBN (nucleic acid sequence based amplification) method, the TMA (transcription-mediated amplification) method and the like] is known, however, in the case of such a method, it is difficult to amplify a single-stranded DNA fragment which is highly identical to a template DNA. For example, in the case of the PCR method, it is known that a mutation is introduced with a probability of one in several hundreds bases (Takagi M, Nishioka M, Kakihara H, Kitabayashi M, Inoue H, Kawakami H, Oka M and Imanaka T. Characterization of DNA polymerase from Pyrococcus sp. strain KOD1 and its application to PCR, Appl Environ Microbiol. 1997; 63:4504-4510). As for such an "unexpected mutation" in the single-stranded DNA fragment, there is a risk that a new mutation may be introduced into a normal base when the single-stranded DNA fragment is used, for example, for correcting a disease gene, therefore it is not preferred. Further, in the case of an E. coli plasmid, its insert (a double-stranded DNA fragment) can be amplified with 10⁸-fold greater accuracy compared with the PCR method (see Drake JW. Comparative rates of spontaneous mutation. Nature. 1969; 221:1132). However, in the case where the amplified double-stranded DNA fragment is denatured to form a single-stranded DNA fragments, it is difficult to separate the sense strand and the antisense strand. Accordingly, particularly in the case where a sense single-stranded DNA fragment is allowed to act on a target DNA sequence, preparation of a single-stranded DNA fragment from a plasmid DNA is not preferred (such a disadvantage also applies to the case where a single-stranded DNA fragment is prepared from a PCR product (double-stranded DNA fragment)).

On the other hand, the method of this invention can also be used in order to create an unnatural cell or individual animal (such a cell or individual animal is useful, for example, as a compound screening system utilizing the difference in sensitivity to a specific compound or the like from a normal form or as a disease model cell or a disease model animal or the like) by introducing a mutation into a normal genomic gene sequence in a cell. Such a single-stranded DNA fragment for converting one or more bases in a normal target DNA sequence (that is, for artificial mutation introduction) can be prepared from a single-stranded circular DNA (for example, a phagemid DNA or the like) prepared by a method using a commercially available mutagenesis kit or the like or a known method such as the PCR method for mutagenesis.

In order to introduce the single-stranded DNA fragment prepared from a single-stranded circular DNA as described above into a cell, a known technique such as the calcium phosphate method, a method using liposome or erythrocyte ghost, the electroporation method or the microinjection method using a glass pipette can be used.

Further, the single-stranded DNA fragment can be introduced into a cell present in an organism. In this case, a method of administering the single-stranded DNA fragment in an organism in a state where it is mixed with an appropriate solvent can be adopted. Alternatively, the single-stranded DNA fragment is embedded in a hollow nanoparticle, liposome or the like each of which presents a molecule recognizing an organism, and the resulting matter may be introduced into the organism. Further, the electroporation method can also be adopted.

Further, in the case where a target DNA sequence to be involved is a "causative gene for a disease" such as a gene causing a disease due to a conversion mutation of one or more bases of the target sequence, by using this method of introducing a single-stranded DNA fragment into a cell present in an organism, it becomes possible to correct the base conversion mutation of the causative gene (the fifth invention). Further, by forming such a single-stranded DNA fragment in the "form that can be introduced into a cell" as described above, a therapeutic agent for a disease caused by a base conversion mutation in a gene can be formed (the fourth invention).

Incidentally, in the mutation known as the cause of a genetic disease, not only a causative gene requiring conversion of one base for correction, but a causative gene such that an accurate and long base sequence is required in the single-stranded DNA used for correction, for example, a long-strand insertion mutation of CAG repeat observed in Huntington's disease (McMurray CT. Huntington's disease: new hope for therapeutics. Trends Nerosci. 2001; 24:S32-S38), or a long-strand deletion mutation in the dystrophin gene of muscular dystrophy (Forrest SM, Cross GS, Speer A, Gardner-Medwin D, Burns J and Davies KE. Preferential deletion of exons in Duchenne and Becker muscular dystrophines. Nature. 1987; 329:638-640. 19. Den Dunnen JT, Bakker E, Breteler EG, Pearson PL and van-Ommen GJ. Direct detection of more than 50% of the Duchenne muscular dystrophy mutations by field invasion gels. Nature. 1987; 329:640-642) is also included. For such a mutated gene, the therapeutic method and the therapeutic agent of this invention are effective.

The second invention of this application is a "cell" in which one or more bases or a base sequence in a target DNA sequence have been converted to another base or base sequence by the method of the above-mentioned first invention. The type of the cell is not particularly limited, and a prokaryotic cell such as E. coli or Bacillus subtilis, a eucaryotic cell such as yeast, an insect cell, an animal cell or a plant cell or the like can be used as a subject. The cell prepared as described above is, for example, a cell in which conversion of one or more bases have been introduced into its functional gene sequence, and can be used as an in vitro screening material for, for example, an agent, a biologically active substance, a cytotoxic substance as a cell in which a specific function has been deleted or enhanced. Further, for a cell to be used in a bioreactor or fermentation engineering or the like, it can be used for deleting a specific cellular function that is "disadvantageous" in its use.

The third invention of this application is an individual organism which retains the cell of the above-mentioned second invention in the body, and particularly, it is a multicellular organism such as an animal cell or a plant cell. Further, this individual organism may be an individual organism in which the cell of the above-mentioned second invention (the cell subjected to in vitro base conversion) has been transplanted in the body, or an individual organism in which one or more bases in a target DNA sequence in a cell of the body have been converted to another base or base sequence as a result of introducing the single-stranded DNA fragment into the body. In the case where the base replacement of the target DNA sequence causes a disease, such an individual organism is useful as a "disease model animal". Further, various genes can be subjected to base conversion, and can also be used for in vivo screening for a pharmaceutical component or a toxic substance or the like.

Hereinafter, the invention of this application will be described in more detail and specifically with reference to Examples, however, the invention of this application is not limited to the following examples.

### Example

In this Example, a plasmid in which a fusion gene (HygEGFP) of a hygromycin resistance gene (Hyg) and a green fluorescent protein (EGFP) gene had been introduced downstream of the promoter of a mammalian cell or E. coli was constructed, and base replacement by a single-stranded DNA fragment was examined. That is, because in pTENHEX, which is a target plasmid, a stop mutation (Stop: TGA) has been introduced at codon 34 of the HygEGFP gene, a normal HygEGFP gene cannot be expressed in a mammalian cell or E. coli. Therefore, it was confirmed by observing the two phenotypes of hygromycin resistance and fluorescence emission of EGFP, whether or not the codon 34 of this mutated HygEGFP gene was corrected to a normal form of Ser (TCA) by introducing a single-stranded DNA fragment (HygEGFP gene) in which the codon 34 is the normal form of Ser (TCA) into a cell.

In the past studies, it is known that when two pairs of DNA having a homologous sequence are interacted to each other, one of the double strands is separated into single strands and the resulting single strands enter into the other double strand (Kowalczykowski SC. Initiation of genetic recombination and recombination-dependent replication, Trend Biochem Sci. 2000; 25:156-165; Baumann P, Bensen FE and West SC. Human Rad51 protein promotes ATP-dependent homologous pairing and strand transfer reactions in vitro. Cell. 1996; 87:757-766). Therefore, assuming that there is a similar mechanism to this in the mechanism of the SFHR method, it is expected that when gene correction is carried out by preparing a single-stranded DNA in advance instead of using a double-stranded DNA, higher gene correction efficiency can be obtained. Accordingly, a circular single-stranded DNA was prepared using a phagemid DNA, which was cut out with a restriction enzyme to form single-stranded DNA fragments (fSense, fAntiS), and the resulting fragments were used.

Incidentally, as for a double-stranded DNA fragment to be used for comparison, a double-stranded DNA fragment was prepared, using a restriction enzyme, from a plasmid replicated in E. coli with 10⁸-fold greater accuracy compared with the PCR method (Drake JW. Nature. 1969; 221:11329), and one obtained by thermal denaturation of the resulting fragment (dsHES) was used.

### (1) Method

### (1-1) Plasmid and phagemid

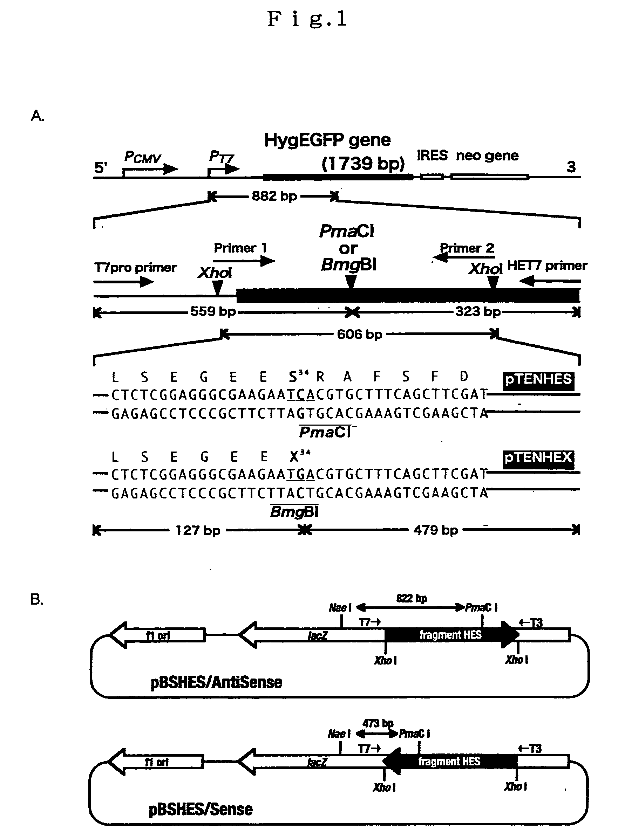

A site-directed mutagenesis reaction was carried out using Altered Sites II in vitro Mutagenesis System (Promega Corp., WI) for pALHE in which a KpnI-SalI fragment of pHygEGFP (CLONTECH Laboratories Inc., CA) had been introduced at the same restriction enzyme sites of pALTER-1 (Promega Corp., WI). In the first reaction, a XhoI site was introduced using an oligonucleotide Xho (5'-cggcacctcgagcacgcggat-3': SEQ ID NO. 1) (pALHEX). At this time, codon 195 was changed from Val to Glu, however, determination of gene correction reaction was not affected. In the second reaction, a PmaCI site, which becomes a gene marker, was introduced at codon 34 of a normal HygEGFP gene using the oligonucleotide Silent (5'-gcgaagaatcacgtgctttca-3': SEQ ID No. 2) (pALHEXP). Further, for pALHEXP, in order to introduce an opal mutation at the codon 34, the oligonucleotide Opal (5'-ggcgaagaatgacgtgctttc-3': SEQ ID No. 3) was used (pALHEXB). In the final reaction, the previously introduced PmaCI site was removed at the same time as the opal mutation, and a BmgBI site as a marker for a mutated form of HygEGFP gene was introduced instead. The sequences of the respective HygEGFP genes were confirmed by a sequence reaction. The BamHI-SalI fragments were purified from pALHEXP and pALHEXB, and introduced at a NcoI-XhoI site present downstream of CMV promoter and T7 promoter of pTriEX-3Neo (Novagen, WI) using NXB linker (Upper: 5'-catggcgatcctcga-3': SEQ ID No. 4, Lower: 5'-gatctcgaggatcgc-3': SEQ ID No. 5) (pTENHES, pTENHEX, Fig. 1A). It was confirmed that the plasmids thus obtained could express a normal form or a mutated form of HygEGFP gene in both cells of E. coli and a mammal, respectively. These plasmids were introduced into an E. coli DH-5α strain, and prepared using Endofree Mega Kit (QIAGEN, CA).

A phagemid was obtained by introducing a XhoI fragment of pTENHES at a XhoI site of pBluescript II SK+ (Fig. 1B). In the thus obtained pBSHES/AntiSense and pBSHES/Sense, the XhoI fragments were inserted in the opposite direction to f1 ori of pBluescript II SK+ (Stratagene, CA), respectively, and a single-stranded circular DNA obtained from this phagemid contains an antisense sequence or a sense sequence of the HygEGFP gene. The insertion direction was confirmed using restriction enzymes, Nael and PmaCI. These phagemids were introduced into a JM105 strain, and the strain was cultured overnight in 2 x YT medium containing ampicillin at 50 µg/ml. Then, a 5 ml portion of the culture was transferred to 500 ml of 2 x YT medium (50 µg/ml Amp), and the culture was incubated at 37°C while vigorously stirring. After 1 hour, kanamycin was added thereto to give a final concentration of 25 µg/ml, and the incubation was restarted and continued. After 23 hours, phage and E. coli were separated by centrifugation (2.15 x 10³ g, 15 min). To the phage contained in the supernatant, 75 ml of 20% PEG 6000/2.5 M NaCl solution was added, and the mixture was let stand overnight at 4°C. After the phage was precipitated by centrifugation (18.8 x 10³ g, 20 min), it was suspended in 20 ml of 0.3 M AcONa/1 mM EDTA, and then, a circular single-stranded DNA was recovered with phenol, phenol/chloroform (1/1) and chloroform. To the DNA solution, 25 ml of EtOH was added, and the mixture was let stand at room temperature for 15 minutes. Then, DNA was precipitated by centrifugation and dissolved in 500 µl of H₂O.

### (1-2) Preparation of fragment

Digestion was carried out with 2.5 U of restriction enzyme XhoI per 1 µg of pTENHES, and a 606-bp fragment was separated with a 3.5% low-melting agarose gel. Then, the fragment was recovered by phenol/chloroform extraction. In addition, by using 5 U of XhoI per 1 pmol (1.1 µg) of the single-stranded pBSHES/AntiSense or pBSHES/Sense, and in the same manner as a double strand, a 606-nt fragment was recovered. In the restriction enzyme treatment of the circular single-stranded DNA, a double strand is formed in the vicinity of the XhoI site, and therefore, oligonucleotides homologous with the XhoI sites at the 5' and 3' sides of pBSHES/AntiSense, AS5' (5'-cccccctcgagatcccc-3': SEQ ID No. 6) and AS3' (5'-cggcacctcgaggtcgac-3': SEQ ID No. 7), or oligonucleotides homologous with the XhoI sites at the 5' and 3' sides of pBSHES/Sense, S5' (5'-cccccctcgaggtgccg-3': SEQ ID No. 8) and S3' (5'-ggggctctgaggtcgac-3': SEQ ID No. 9) were added to a solution for restriction enzyme reaction in an amount of 5 molar equivalent of the circular single strand.

The recovered fragments were purified by gel filtration (NAP5 columns, Amersham Biosciences Ltd., Buckinghamshire, England), respectively, and the purification degree thereof expressed by the A₂₆₀/A₂₈₀ ratio was 1.8 or more. The concentration was calculated by assuming 1.0 OD₂₆₀ to be 50 µg for the double-stranded DNA, and 40 µg for the single-stranded DNA.

### (1-3) Gene introduction

On the day before transfection, 3 x 10⁵ CHO-K1 cells suspended in 4 ml of D'MEM/F12 (Invitrogen, Corp., Carlsbad, CA) containing 10% FBS were seeded on a 6-cm dish, and the dish was incubated at 37°C in a 5% CO₂ atmosphere. To 2.5, 5, 10 nmol (0.47, 0.94, 1.9 µg, respectively) of single-stranded DNA or 10 nmol (3.8 µg) of double-stranded DNA, all of which had been subjected to a thermal denaturation treatment (at 98°C for 5 min, then at 0°C for 5 min) in advance, 149 µl of D'MEM/F12 containing 72 µg of PLUS reagent (Invitrogen, Corp., Carlsbad, CA), and 25 fmol (125 ng) of pTENHEX were added. Further, because it has been reported that the difference in N/P ratio significantly affects the gene correction efficiency (Sangiuolo F, Bruscia E, Serafino A, Nardone AM, Bonifazi E, Lais M, Gruenert DC and Novelli G. In vitro correction of cystic fibrosis epithelial cell lines by small fragment homologous replacement (SFHR) technique. BMC Med Genet. 2002; 3:8), in order to make the DNA amount in all the samples equal (total of 4 µg), pALTER-Ex2 (Promega Corp., WI) which does not contain Amp resistance gene, was added in an appropriate amount. After the mixture was left at room temperature for 20 minutes, 141 µl of D'MEM/F12 containing 32 µg of LipofectAMINE reagent (Invitrogen, Corp., Carlsbad, CA) was added. Then, the mixture was left at room temperature for an additional 20 minutes, and immediately before the treatment of cells was carried out, 250 µl of D'MEM/F12 was added. After the cells were washed with 4 ml of D'MEM/F12, the medium was replaced with 2 ml of D'MEM/F12. Then, the total volume of the DNA solution prepared previously was added thereto, and the mixture was incubated at 37°C in a 5% CO₂ atmosphere. After 6 hours, the reaction solution was replaced with 4 ml of D'MEM/F12 containing 10% FBS, thereby finishing the introduction reaction. The cells were further cultured for 42 hours and then, subjected to a trypsin treatment. Then, the cells were washed with 1 ml of PBS, and collected by centrifugation. The collected cells were suspended in 100 µl of TEG (25 mM Tris-HCl, 10 mM EDTA, 50 mM Glc, pH 8.0), and 200 µl of 0.2 N NaOH/1% SDS was added thereto, and the mixture was gently stirred and left at room temperature for 5 minutes. Further, 150 µl of 8 M AcONH₄ was added thereto, and the mixture was cooled at 4°C for 15 minutes. After the mixture was centrifuged, the plasmid DNA was recovered from the supernatant by isoPrOH precipitation. The plasmid DNA was dissolved in 20 µl of H₂O, and stored at -20°C.

### (1-4) Determination of gene correction efficiency

DH-5a which had been cultured in 1.4 ml of LB medium from the previous day was transferred in an amount of 125 µl per one sample to 12.5 ml of fresh LB medium and cultured for an additional 4 hours. The E. coli was washed with 7.5, 1, and 0.2 ml of cooled H₂O, and finally, cooled H₂O was added thereto such that the final volume was about 100 µl to resuspend the E. coli. The resulting suspension was transferred to a cuvette with a 0.1-cm gap along with 4 µl of pDNA, and electroporation was carried out using Gene Pulser II (Bio-Rad Laboratories Inc. CA) under the conditions of 4°C, 1.8 kV, 25 µF and 200 Ω. DH-5α into which a plasmid had been introduced was cultured in 1 ml of SOC medium for 1 hour, and the culture was diluted with LB medium supplemented with 50 µg/ml Amp, and incubated overnight. At this time, a portion of DH-5α was taken and seeded on LB agar medium, and the electroporation efficiency was determined based on the number of colonies, however, a significant difference was not observed. DH-5α obtained on the next day was recovered by centrifugation, and resuspended in 100 µl of TEG. To the suspension, 200 µl of 0.2 N NaOH/1% SDS was added and the mixture was gently stirred. Then, 150 µl of Sol III (3 M potassium acetate, 11.5% acetic acid) was further added thereto, and the mixture was left on ice for 5 minutes. The supernatant obtained by centrifugation was treated with phenol/ chloroform (1/1), and plasmid DNA was recovered by ethanol precipitation. The recovered plasmid DNA was dissolved in 20 µl of H₂O. A 1 to 2 µl portion of the DNA solution was used for introduction into an E. coli BL21 (DE3) strain by electroporation. At this time, in the same manner as DH-5α, transformation of BL21 (DE3) was carried out.

After the transformation, 1 ml of SOC medium was added, and incubation was carried out at 37°C for 1 hour. A 50 µl portion of the culture was seeded in 1 ml of LB medium containing 50 µg/ml Amp and 10 µM IPTG and incubation was carried out at 37°C for 3 hours. After the incubation, the culture diluted 1 to 10-fold was seeded on LB agar medium supplemented with 75 µg/ml hygromycin B (Hyg75: 50 µg/ml Amp, 10 µM IPTG), and the culture diluted 100 to 1000-fold was seeded on LB agar medium free of hygromycin B (Hyg0: 50 µg/ml Amp, 10 µM IPTG) in equal amounts, and incubation was carried out at 37°C. After 12 to 24 hours, the number of colonies generated on Hyg0 was counted. In addition, the colonies generated on Hyg75 after 26 to 48 hours were analyzed using FLA2000G (FUJI PHOTO FILM Co., Ltd., Kanagawa, Japan), and colonies emitting fluorescence of EGFP (Ex. 473 nm, Em. 520 nm) were counted. The number of colonies obtained on Hyg0 was made the denominator, and the number of EGFP-fluorescence- positive colonies obtained on Hyg75 was made the numerator, and the gene correction efficiency was thus calculated.

### (1-5) Confirmation of genotype

Some of the EGFP-positive colonies obtained on the Hyg75 plate were selected, and a part thereof was suspended in 20 µl of H₂O, and then the suspension was subjected to a heat treatment at 98°C for 5 minutes. Insoluble components of the bacteria were separated as a pellet by centrifugation, and a 2 µl portion of the supernatant was used as a template for PCR which was carried out with rTaq DNA polymerase (TOYOBO Co., Ltd., Osaka, Japan). At this time, as the primers, T7pro (5'-taatacgactcactataggg-3': SEQ ID NO. 10) and HET7 (5'-atcgcctcgctccagtcaat-3': SEQ ID NO. 11) were used. The thus obtained PCR product was further treated with PmaCI, which is a marker of the normal form of HygEGFP gene, and was used in a sequencing reaction with ABI PRISM BigDye Terminator v3.1 Cycle Sequencing Kit (Applied Biosystems, Foster City, CA).

### (2) Results

### (2-1) Evaluation system of gene correction reaction

In pTENHES and pTENHEX (Fig. 1A), the normal form and mutated form of HygEGFP genes have been integrated, respectively, and the respective codons 34 are Ser (TCA: PmaCI) and Stop (TGA: BmgBI). Further, in the phagemid vectors for preparing a single strand, pBSHES/AntiSense and pBSHEX/Sense (Fig. 1B), a part of the normal form of HygEGFP gene obtained by treating pTENHES with XhoI has been integrated. When they are formed into circular single strands, they encode an antisense sequence and a sense sequence, respectively.

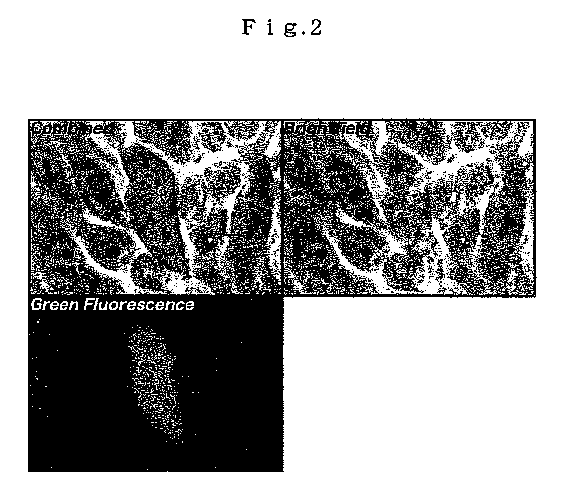

Hereinafter the DNA fragments obtained by treating pTENHES and single-stranded circular pBSHES/AntiSense and pBSHES/Sense with XhoI are referred to as dsHES, fAntiS and fSense, respectively. The target plasmid pTENHEX was introduced into a CHO-K1 cell along with dsHES, fAntiS or fSense. Because in this plasmid, the mutated form of HygEGFP gene is regulated by CMV promoter for the expression in a mammalian cell and T7 promoter for the expression in E. coli, when the repair reaction succeeded, fluorescence of EGFP is observed in both cells (Fig. 2). Further, the cells acquire hygromycin B resistance, therefore, a corrected gene can be easily isolated (Fig. 3).

### (2-2) Gene correction reaction

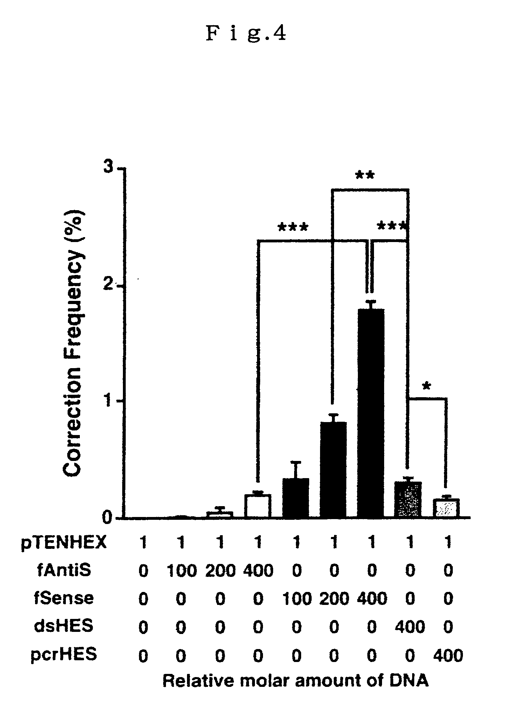

Into a CHO-K1 cell, pTENHEX, which is a target, was introduced along with a DNA fragment obtained by the treatment with XhoI. At this time, because dsHES was a double strand, in the same manner as the SFHR method, it was thermally denatured just before it was introduced into a cell. Further, although fAntiS and fSense were a single-stranded DNA, in order to elucidate their intramolecular conformation and to perform comparison with dsHES, a thermal denaturation treatment was carried out in the same manner as dsHES, and introduction into a cell was carried out. When the cell was observed using a fluorescence microscope at 48 hours after the initiation of introduction, it was observed that the corrected HygEGFP gene was expressed in the CHO-K1 cell (Fig. 2). Plasmids were recovered from these cells, colonies with hygromycin B resistance and indicating EGFP-positive (Fig. 3), both of which are acquired by transformation into BL21 (DE3), were identified, and a gene correction efficiency was calculated. When 10 nmol of DNA fragment, which corresponds to 400-fold molar ratio relative to pTENHEX, was treated, in the case of dsHES, which is a double-stranded DNA fragment, the correction efficiency was 0.43%, which is equivalent to that of the conventional SFHR method (Fig. 4). On the other hand, although fAntiS, which is a single-stranded DNA fragment, did not show a gene correction efficiency beyond that of dsHES (0.15%), in the case of treating fSense, a gene correction efficiency of 2.0%, which is about 5 times as high as that of dsHES, was shown (Fig. 4).

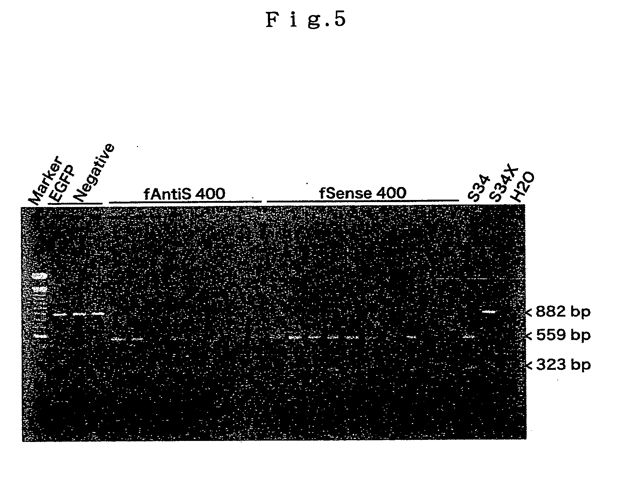

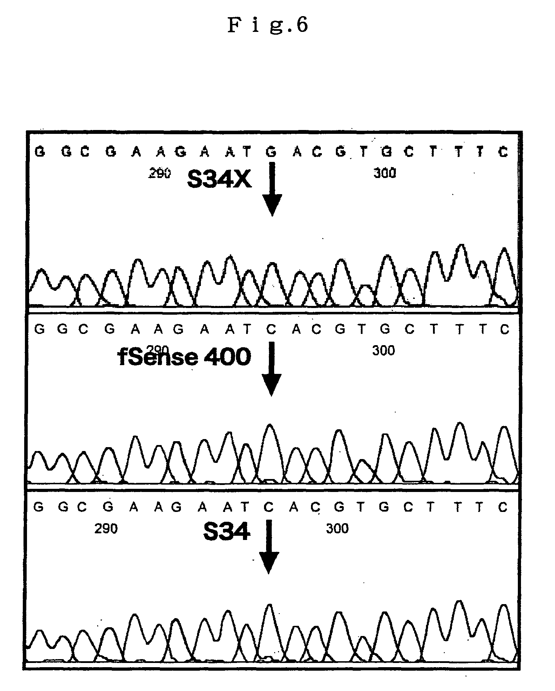

Some of the EGFP-positive colonies obtained by this analysis were selected, and their gene sequences were confirmed with a restriction enzyme, PmaCI (fig. 5) and by a sequencing reaction (Fig. 6). As a result, sequence conversion at codon 34 from Stop to Ser (TGA → TCA) was confirmed. Further, because other sequence conversion was not observed, sequence specificity in this method was confirmed.

### Comparative example

For pTENHEX (a plasmid DNA retaining a HygEGFP gene in which a stop mutation (Stop: TGA) had been introduced at codon 34) used in Example, by performing the conventional SFHR method using a PCR product, a correction efficiency of a mutated HygEGFP gene was examined in the same manner as in Example.

A PCR product (pcrHES) was amplified using the following primer 1 and Taq polymerase (manufactured by TOYOBO Co., Ltd.), and the 3'-end thereof was blunted using Blunting High Kit (manufactured by TOYOBO Co., Ltd.), and produced by a 3.5% low-melting agarose gel electrophoresis in the same manner as dsHES in Example.
Primer 1: 5'-gagatccccggagccg-3' (SEQ ID No. 10)
Primer 2: 5'-gaggtgccggacttcgg-3' (SEQ ID No. 11)

The results are as shown in Fig. 3 and Fig. 4. As for the PCR product (pcrHES), the effect of the cell acquiring hygromycin B resistance trait was low compared with the single-stranded sense DNA fragment (fSense) (Fig. 3), and the conversion efficiency was 0.16%. This value is not more than half the conversion efficiency of dsHES (0.43%), and not more than one-tenth the conversion efficiency of the single-stranded sense DNA fragment (fSense) (2.0%).

### Industrial Applicability

As described in detail above, according to the invention of this application, a method of converting a specific base or base sequence in a DNA sequence in a cell to another base or base sequence with high efficiency is provided. This enables efficient correction of a mutated site of such as a disease gene.

## Claims

1. A base conversion method of a DNA sequence, which is a method of converting one or more bases in a target DNA sequence in a cell, **characterized by** introducing a single-stranded DNA fragment having 300 to 3,000 bases which is prepared from a single-stranded circular DNA, is homologous with the target DNA sequence, and contains the base(s) to be converted, into a cell.

2. The method according to claim 1, wherein the single-stranded circular DNA is a phagemid DNA.

3. The method according to claim 1 or 2, wherein the single-stranded DNA fragment is homologous with a sense strand of the target DNA sequence.

4. The method according to any one of claims 1 to 3, wherein the target DNA sequence in the cell is a DNA sequence causing a disease due to the one or more bases.

5. The method according to any one of claims 1 to 4, wherein one or more bases in a target DNA sequence in a cell of an organism are converted.

6. A cell in which one or more bases in a target DNA sequence have been converted by the method according to any one of claims 1 to 5.

7. An individual organism which retains the cell according to claim 6 in the body.

8. A therapeutic agent, which is an agent for treating a disease caused by conversion of one or more bases in a target DNA sequence, **characterized in that** a single-stranded DNA fragment having 300 to 3,000 bases which is prepared from a single-stranded circular DNA is homologous with the target DNA sequence and contains the base(s) to be converted, and has a form that can be introduced into a cell.

9. The therapeutic agent according to claim 8, wherein the single-stranded circular DNA is a phagemid DNA.

10. A therapeutic method, which is a method of treating a disease caused by conversion of one or more bases in a target DNA sequence, **characterized by** introducing a single-stranded DNA fragment having 300 to 3,000 bases which is prepared from a single-stranded circular DNA, is homologous with the target DNA sequence, and contains the base(s) to be converted, into a cell.

11. The therapeutic method according to claim 10, wherein the single-stranded circular DNA is a phagemid DNA.


SEQUENCE LISTING

<110> Japan Science and Technology Agency

<120> A Method of Base Modification for DNA Sequence

<130> 05F003PCT

<150> JP 2004-34019
<151> 2004-02-10

<160> 11

<170> PatentIn version 3.1

<210> 1
<211> 21
<212> DNA
<213> Artificia

<220>
<223> Description of artificial sequence: Synthetic oligonucleotide
<400> 1
cggcacctcg agcacgcgga t                                    21

<210> 2
<211> 21

<212> DNA
<213> Artificia

<220>
<223> Description of artificial sequence: Synthetic oligonucleotide
<400> 2
gcgaagaatc acgtgctttc a 21

<210> 3
<211> 21
<212> DNA
<213> Artificia

<220>
<223> Description of artificial sequence: Synthetic oligonucleotide
<400> 3
ggcgaagaat gacgtgcttt c 21

<210> 4
<211> 15
<212> DNA
<213> Artificia

<220>
<223> Description of artificial sequence: Synthetic oligonucleotide
<400> 4

catggcgatc ctcga 15

<210> 5
<211> 15
<212> DNA
<213> Artificia

<220>
<223> Description of artificial sequence: Synthetic oligonucleotide
<400> 5
gatctcgagg atcgc 15

<210> 6
<211> 17
<212> DNA
<213> Artificia

<220>
<223> Description of artificial sequence: Synthetic oligonucleotide
<400> 6
   cccccctcga gatcccc 17

<210> 7
<211> 18
<212> DNA

<213> Artificia

<220>
<223> Description of artificial sequence: Synthetic oligonucleotide
<400> 7
cggcacctcg aggtcgac 18

<210> 8
<211> 17
<212> DNA
<213> Artificia

<220>
<223> Description of artificial sequence: Synthetic oligonucleotide
<400> 8
ccccccctcga ggtgccg 17

<210> 9
<211> 18
<212> DNA
<213> Artificia

<220>
<223> Description of artificial sequence: Synthetic oligonucleotide
<400> 9
ggggctctcg aggtcgac 18

<210> 10

<211> 16

<212> DNA

<213> Artificia

<220>

<223> Description of artificial sequence: Synthetic oligonucleotide

<400> 10

gagatccccg gagccg 16

<210> 11

<211> 17

<212> DNA

<213> Artificia

<220>

<223> Description of artificial sequence: Synthetic oligonucleotide

<400> 11

gaggtgccgg acttcgg 17